Europäisches Patentamt

(19) European Patent Office

Office européen des brevets

(11) Publication number: **0 039 243**
**B1**

(12) **EUROPEAN PATENT SPECIFICATION**

(45) Date of publication of patent specification: 02.10.85

(51) Int. Cl.⁴: **A 61 B 5/14**

(21) Application number: 81301887.6

(22) Date of filing: 29.04.81

(54) Transcutaneous carbon dioxide measuring assembly.

(30) Priority: 29.04.80 JP 57594/80
29.04.80 JP 57595/80
29.04.80 JP 57596/80
29.04.80 JP 57597/80

(43) Date of publication of application:
04.11.81 Bulletin 81/44

(45) Publication of the grant of the patent:
02.10.85 Bulletin 85/40

(84) Designated Contracting States:
CH DE FR GB IT LI SE

(56) References cited:
DE-A-2 849 973
FR-A-2 276 586
FR-A-2 376 412
US-A-3 415 730
US-A-3 694 734

JOURNAL OF ELECTROCHEMICAL SOCIETY,
vol. 126, no. 5, May 1979, pages 793-795,
Princeton, U.S.A., T.A. FJELDLY et al.: "Solid-
state ion-selective electrodes with integrated
electronics"

(73) Proprietor: **SUMITOMO ELECTRIC INDUSTRIES
LIMITED**
**No. 15, Kitahama 5-chome Higashi-ku
Osaka-shi Osaka-fu (JP)**

(72) Inventor: **Fukai, Tamotsu Osaka Works of
Sumitomo Electric
Industries, Ltd. No. 1-3, Shimaya 1-chome
Konohana-ku, Osaka (JP)**
Inventor: **Ohkawa, Shinichi Osaka Works of
Sumitomo Electric
Industries, Ltd. No. 1-3, Shimaya 1-chome
Konohana-ku, Osaka (JP)**
Inventor: **Hiramoto, Junichi Osaka Works of
Sumitomo Electric
Industries, Ltd. No. 1-3, Shimaya 1-chome
Konohana-ku, Osaka (JP)**

(74) Representative: **Allard, Susan Joyce et al
BOULT, WADE & TENNANT 27 Furnival Street
London EC4A 1PQ (GB)**

Courier Press, Leamington Spa, England.

# 0 039 243

## Description

The present invention relates to a $CO_2$ measuring assembly suitable for the transcutaneous measurement of the concentration of carbon dioxide (or of the partial pressure thereof) in the tissue metabolism or in the blood.

Detecting the concentration of $CO_2$ in the blood is extremely important for clinical tests for studying breathing and metabolism and for determining the approximate pH value of the blood of the patients. A method of direct measurement by withdrawal of the blood from an artery for detecting the $CO_2$ concentration in the blood is known. This method was, however, not suitable for continuous measurement and caused pain to the patient. This method was particularly not suitable for the neonatal and premature babies because of the possible risks when collecting blood from the patients.

A transcutaneous measuring electrode assembly is also known wherein carbon dioxide which diffuses from the blood or tissue is trapped at the skin surface and chronological continuous measurement without causing pain to the patient is made possible.

The present invention relates to an improvement in the transcutaneous measurement of carbon dioxide in the blood, and, more particularly, to the improvement made to the structure of the glass electrode, to the heating structure and to the signal to noise ratio of the input signal.

According to the first aspect of the present invention there is provided a transcutaneous carbon dioxide measuring assembly which comprises an electrode holder having a glass electrode with an $H^+$ ion sensitive glass membrane at the end of a tubular member made of an insulating material, and an external reference electrode located around the circumference of the glass electrode, a membrane holder located around the circumference of the reference electrode having a $CO_2$ permeable polymer membrane disposed at one end thereof, and a membrane fixing means having an aperture through which the gas to be measured is passed to the glass electrode, the membrane fixing means pressing the polymer membrane of the membrane holder onto the glass membrane via a $CO_2$ responsive electrolyte film, the electrode holder, membrane holder and membrane fixing means being three detachable components which are joined to form the said assembly, characterized in that a conductive coating is formed on the inner surface of the glass membrane and a heater member comprising a heater and a heat sensitive element which controls the heater to maintain a predetermined temperature is disposed inside the glass electrode.

According to a second aspect of the invention there is provided a transcutaneous carbon dioxide measuring assembly which comprises an electrode holder having a glass electrode with a $H^+$ ion sensitive glass membrane at the end of the rubular member made of an insulating material, and an external reference electrode located around the circumference of the glass electrode, a membrane holder located around the circumference of the reference electrode having a $CO_2$ permeable polymer membrane disposed at one end thereof, and a membrane fixing means having an aperture through which the gas to be measured is passed to the gas electrode, the membrane fixing means pressing the polymer membrane of the membrane holder onto the glass membrane via a $CO_2$ responsive electrolyte film, the electrode holder, membrane holder and membrane fixing means being three detachable components which are joined to form the said assembly, characterized in that a conductive coating is formed on the inner surface of the glass membrane and a preamplifier is connected to the external reference electrode and to the main measuring device.

The conductive coating is preferably a metal or metallic salt which may be formed by vacuum deposition on the inner surface of an H ion responsive glass membrane comprising a glass electrode, a lead wire for the glass electrode being connected to the metal or metallic salt coating layer, and the glass membrane also being reinforced by a resin such as silicone rubber inside the glass container forming the glass electrode. The carbon dioxide measuring electrode of the assembly of the present invention does not require such electrolyte filled in the glass container of the prior art assembly, and is free from all the problems and difficulties mentioned above. Accordingly, the present invention provides a transcutaneous carbon dioxide measuring assembly which is stable, robust and efficient to operate.

The temperature stabilized glass electrode of the invention limits the heating area to the proximity of the measuring portion and reduces the skin area which is to be heated, thereby eliminating further the possibility of neonatals suffering from burns.

In the conventional assembly, the input impedance to the preamplifier has to be raised because of an extremely high impedance between the electrodes, and the preamplifier of the measurement device provided at a distance from the electrode assembly sensor magnetic induction suffers from electro-static and electro-magnetic induction. However, according to the present invention, the preamplifier contained in the glass electrode is connected to the measurement device with a low output impedance of the preamplifier. Thus, the difficulties concerning the noise caused by the high input impedance of the preamplifiers are removed. Since the preamplifier is contained in the thermostat glass electrode, fluctuations in the preamplifier gains and drifts in the offset based on the temperature changes are extremely small.

The present invention will be further described with reference to the accompanying drawings, in which:—

Figure 1 is a cross sectional view of a conventional carbon dioxide measuring electrode assembly;

2

Figure 2 is a cross sectional view of a carbon dioxide measuring assembly in accordance with a first embodiment of the invention;

Figure 3 is a cross sectional view of a carbon dioxide measuring assembly in accordance with a second embodiment of the invention; and

Figure 4 is a schematic diagram of the circuit for the preamplifier of Figure 3.

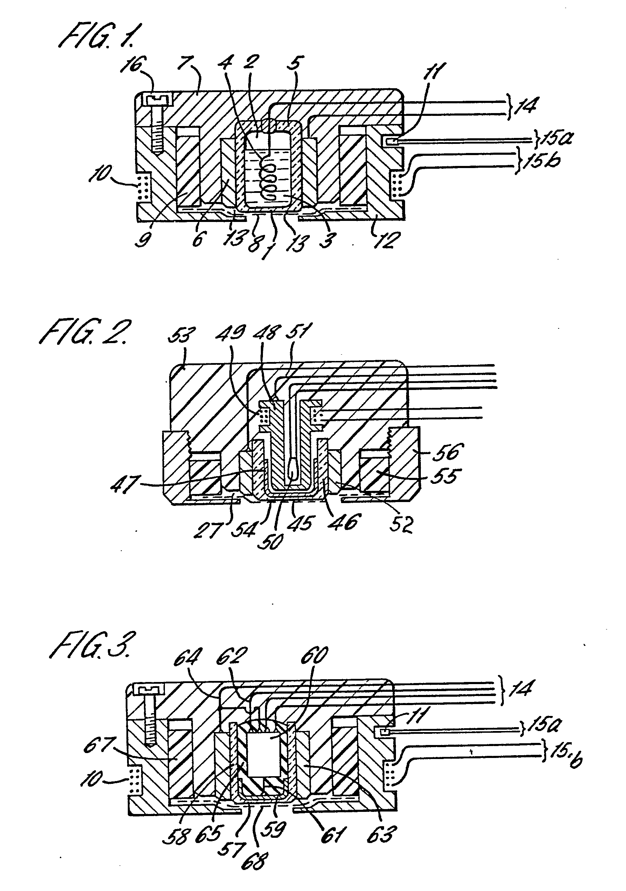

Figure 1 is a cross sectional view showing the basic structure of a conventional type carbon dioxide measuring electrode assembly.

The assembly shown in Figure 1 comprises three separate members which are a top cover 7 centrally locating a glass electrode 5 comprising a glass container 2 having an $H^+$ ion sensitive (pH responsive) glass membrane 1 at the bottom thereof and containing a buffer electrolyte 3 mainly composed of an aqueous solution of KCl or NaCl and a silver wire 4 immersed therein the surface of which has been converted into silver chloride, and having an external reference electrode 6 made of silver and silver chloride located around the glass electrode 5, a membrane holder 9 stretching an electrode membrane 8 at the end portion of the holder 9, and a heating member 12 having a heater means 10 embedded therearound and a heat sensitive element 11 embedded therein, the membrane holder 9 being detachably retained between the top cover 7 and the heating member 12 by the screws 16.

Between the electrode membrane 8 and the glass membrane 1 are the electrolyte 13 comprising an aqueous solution of $NaHCO_3$, NaCl or KCl, and a spacer made of paper or nylon, etc. The reference number 14 denotes a lead wire from the electrodes, 15a and a lead wire from the heat sensitive element 11, and 15b a feed wire to the heater 10. The electrode assembly having an electrolyte contained in the glass electrode as mentioned above as the following defects:

(i) When the assembly is knocked over or is inverted by the movement of a patient, the electrolyte 3 touched accidentally moves away from the glass membrane 1 and silver wire 4 is thus electrically disconnected from the glass membrane 1;

(ii) The electrolyte is accidentally frozen during preservation or transportation in a very cold climate and this causes the glass electrode to break;

(iii) When the temperature of the electrode 5 assembly is elevated during measurement, a portion of the electrolyte 3 vaporizes and forms dew in the glass container 2 and causes abnormalities in the measurement;

(iv) The electrolyte of the glass electrode 5 accidentally leaks and causes the insulation of the electrode to deteriorate;

(v) The glass membrane is susceptible to being broken;

(vi) The temperature of the glass electrode affects its electro motive force so that it is necessary to maintain the temperature fluctuation of the electrode within 0.2°C. However, it is difficult to maintain the temperature of the electrode so stable;

(vii) The conventional type electrode assembly has two separate components of a top cover 7 having a glass electrode and an external reference electrode therearound, and a heating member 12 having a heater 10 and a heat sensitive element 11. Therefore, it is necessary for lead wires 14, lead wires 15a and feed wire 15b to be connected to the top cover and the heating member 12 respectively. Because of the construction having these separated lead wires, it is not possible to fix the heating member 12 and the top cover 7 by way of a screw means, but the bolts 16 must be used instead. As three bolts do not uniformly tighten the top cover and the heater fixed to each other at the times when the membrane holder is changed, the measured values fluctuated. Since the outer diameter of the electrode assembly is as small as 18 mm, a tiny screw of 1 mm $\phi$ must be used. Thus, the replacement of the membrane holder is extremely difficult; and

(viii) In conventional electrode assemblies, however, a pH responsive glass membrane placed on the bottom of the glass electrode shows an extremely high impedance such as $10^9$ ohm to $10^{10}$ ohm, so that the lead wires 14 tend to take up noises caused by electro static and/or electro-magnetic induction.

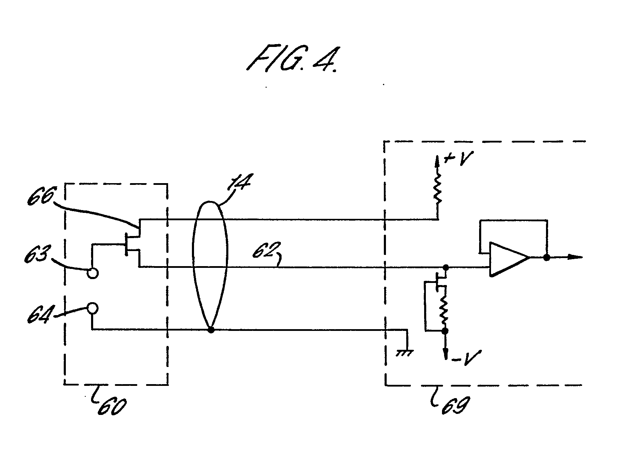

Figure 2 shows a cross sectional view of a transcutaneous $CO_2$ measurement sensor according to the first embodiment of the present invention. Referring to Figure 2, an $H^+$ ion responsive glass membrane 45 is attached to the end of an annular member 46 made of an insulating material such as glass, ceramic, plastic or rubber by fusing or with an adhesive, to form a glass electrode. A conductive coating 47 of a metal such as Au, Pt, Ag, etc., or a metallic salt such as AgCl, etc is formed on the inner surface of the glass membrane 45 by vacuum evaporation or ion plating. The metal coating may also be formed on the inner surface of the glass container by thermally fusing a metal or a metallic salt which has a lower melting point than that of the glass membrane. A particularly important component 48 of the present invention, namely a heater member made of a metal such as silver, copper, aluminium, etc. and having an excellent heat conductivity, houses a heater 49 and a heat sensitive element 50 and is controlled at a desired constant temperature. Figure 2 shows a construction where excellent electrical and thermal conduction may be maintained since the heater member adheres closely to the conductive coating 47 formed on the inner surface of the glass membrane 45. Accordingly, the conductive membrane 45 and the heater member 48 are joined together with a conductive adhesive agent, and the electrical potential of the conductive coating 47 is taken out by lead wire 51.

It is naturally possible to use heater member 48, the surface of which has been insulated, to connect the lead wire 51 to the conductive coating 47. An annular shape external reference electrode 52 comprising silver/silver chloride is provided around the outer circumference of the glass electrode.

3

The glass electrode having a heating mechanism and the external electrode are fixed to an electrode holder 53 made of an insulating material, such as a plastics material or rubber. A membrane holder 55 has a $CO_2$ transmittant and hydrophobic polymer film such as Teflon[R], silicone rubber, etc., attached to the end thereof and it is disposable along with the polymer membrane 54. Between the glass membrane 45 and the polymer membrane 54 there is placed, as in the conventional art, an aqueous solution containing KCl, $NaHCO_3$, etc, as the $CO_2$ responsive electrolyte (of which the pH value varies depending on the $CO_2$ concentration). A pressure plate 56 is provided to press the polymer membrane 54 of the membrane holder 55 against the glass membrane by squeezing a film of the electrolyte 27. The pressure plate is removably screwed to the electrode holder 53.

A specific example of this embodiment of the present invention is described in further detail. The glass membrane 45 composed of 25 wt% $Na_2O$, 10 wt% of CaO and 65 wt% of $SiO_2$ having a diameter of 8 mm and a thickness of 0.15 mm is fused to the end of a lead glass cylinder by soldering. Inside the glass container platinum is vaccum deposited. A heater member 48 made of copper is adhered to the inner wall of the container using a conductive adhesive agent to make up a glass electrode.

Over the outer circumference of the glass electrode a silver ring the surface of which surface has been electrolyzed in 0.1 N-HCl to transform it into silver chloride is fixed as an external reference electrode 52 using an epoxy resin. As the external electrolyte 27, 0.01 mol NaCl and 0.05 mol $NaHCO_3$ were used. A 20 μm thick Teflon[R] film was used as a polymer membrane.'

The sensor according to the present invention manufactured under the conditions as above mentioned was confirmed to the excellent both in performance and operability as shown in Table 1.

TABLE 1

| Items | Prior art | Present invention |
|---|---|---|
| 1. Time until initial stabilization | 25 min. | 8 min. |
| 2. Response time (Gas) ($CO_2$ 5%→10%) | 90% response 37 sec. | 90% response 36 sec. |
| 3. Gas sensitivity ($CO_2$ 5%→10%) | Potential change 17 mV | Potential change 17 mV |
| 4. Correlation to detected value in blood (correlation coefficient of trans-cutaneously measured value and value found in the blood sample) | 0.98 | 0.98 |
| 5. Performance when the sensor falls | Measurement not possible as the potential is disturbed. Original potential not regained when restored to original position. | Excellent perfor-mance continues without change. |
| 6. Temperature at which sensor becomes destroyed due to freezing. | Glass electrode breaks at −12°C | No change is observed at −20°C. |
| 7. Impact resistance of the glass membrane of glass electrode. | Breaks when dropped from the height of 1.5 m | Does not break even when dropped from the height of 2.0 m. |

Using a sensor of the present invention, it is possible to measure $H^+$ ion concentration changes (pH changes) without using an electrolyte inside the glass electrode so that: (a) it is possible to continue the measurement without impairing the function of the sensor when it becomes inverted or falls due to the movement of the patient; (b) it is possible to avoid problems such as damage to the glass electrode as the electrolyte inside the container becomes frozen in cold climates, (c) there is no abnormal behaviour of the potential due to vaporization and dew concentration of the electrolyte in the hollow portion of the glass

4

electrode when the sensor is heated, (d) there are no difficulties caused by the leakage of electrolyte from the glass electrode, (e) the mechanical strength of the $H^+$ ion sensitive membrane increases since a solid body such as a metal or silicone or epoxy resin is filled inside the glass electrode, thus making the sensor less liable to breakage.

Furthermore, by having the heater body 48 inside the glass electrode:

(1) The electrode reaches the measurement temperature in an extremely short period of time and accurately maintains the said temperature at a constant value thereafter because the pH responsive glass membrane directly contacts the heater member which is made of an excellent heat conductive metal. Therefore, there is no drift in the measurement value seen in the generated potential arising from the temperature variation at the electrode as often has often been observed in conventional products.

(2) As the conventional product has a structure where an annular shaped heating member is attached around the glass electrode, and the diameter of the glass electrode must be made large, the skin temperature of the measurement portion does not rise quickly. Thus, it was impossible to expect a sensor with a good response. According to the present invention, the glass electrode itself heats the skin, and the diameter of the glass electrode may be arbitrarily determined to adapt it to any required performance.

(3) The conventional product provided an annular shaped skin heating member over the outer circumference of the glass electrode, whereby the heated skin area inevitably increased. When the patient suffered a burn the scar was large. However, the product according to the present invention heats the skin only in about the same area as the cross section of the glass electrode. This means that the scar of a burn, is far smaller than those seen in patients for whom the conventional type was used.

(4) Because the temperature effect of the electromotive force of the glass electrode is large, it is necessary to maintain the temperature of the glass electrode constant within $\pm 0.2°C$. The heating mechanism as employed in the present invention sufficiently meets this condition.

As explained hereinabove, the present invention not only removes all the disadvantages of the conventional transcutaneous $CO_2$ sensor, but also offers an excellent transcutaneous $CO_2$ sensor with good performance and ease of operation.

Figure 3 further shows a cross sectional view of a transcutaneous $CO_2$ sensor according to the second embodiment of the present invention. Referring to Figure 3 a pH responsive glass membrane 57 is attached to the bottom of a support tube 58 to form a glass electrode. A conductive coating 59 is formed on the inner surface of the said glass membrane 57. A preamplifier 60 provided inside the glass electrode support tube 58 has a lead wire 61 for taking up the potential which is extremely short and connected to the conductive coating 59. It provides the preamplifier with a lower output impedance and the preamplifier 60 is connected to the main measuring device by lead wire 62. The external reference electrode 63 is disposed In such a way that it encircles the glass electrode, and the potential of the electrode 63 is connected to the preamplifier 60 which is connected to the main measuring device by the lead wire 64. The heat generating member 10 and the heat sensitive element 11 are the same as those used in the conventional art, and the lead wire 15a, the feeder line 15b from these member and element are connected to the main measuring device 69. A disposable membrane holder 67 has a stretched polymer membrane 68 at the end thereof.

A specific example of the second embodiment of the present invention is explained in more detail below.

A pH responsive glass membrane 57 having a composition of 25 mol% $LiO_2$, 8 mol% BaO, and 67 mol% of $SiO_2$ and a thickness of 120 μm was used in the experiment. The glass electrode support tube 58 comprises an insulated epoxy resin tube having an outer diameter of 6 mm, an inner diameter of 5 mm and a height of 8 mm. The membrane 57 was adhered to the bottom of the support tube 58 with a silicone adhesive, and on the inner bottom surface thereof was deposited a 7 μm thick coating of silver by vacuum deposition to form a conductive solid film 59. Metallic case type MOS FET was used as a preamplifier 60 (diameter 4 mm×height 4 mm), and the lead wire 61 for the preamplifier was adhered with a silver powder containing a conductive epoxy resin. A silicone rubber compound which hardened at normal temperatures was filled in the void 65 inside the glass electrode, except for the preamplifier and the lead wire, in order to increase and enhance the mechanical strength and insulating properties thereof.

The circuit for the preamplifier comprises MOS type FET 66 as shown in Figure 4 and the output of the preamplifier is connected to the main measuring device by the lead wire 62. The ground potential terminal of the preamplifier is connected to the external reference electrode 63 which is maintained at the earth potential for shield purposes, and is connected to the ground potential terminal of the main measuring device by the lead wire 14. Table 4 shows the results of the performance comparison of the conventional sensor shown in Figure 1 and the sensor of the present invention shown in Figure 3. In the comparative experiment, poly-tetra-fluoroethylene membrane 13 μm in thickness were used for both the conventional assembly and the assembly of the present invention. An aqueous solution containing 0.005 mol $NaHCO_3$ and 0.02 mol NaCl was used as the electrolyte to be maintained between the polymer membrane and the glass electrode.

# 0 039 243

TABLE 2

| | Conventional | Present Invention |
|---|---|---|
| **Comparison of transcutaneous $CO_2$ measurement assemblies** | | |
| Gas response sensitivity ($CO_2$ 5%—10%) | Potential change, 16 mV | Potential change, 16 mV |
| Noise level | Potential change less than 2.3 mV | Potential change less than 0.1 mV |
| Drift | Potential change, less than 0.4 mV/H | Potential change, less than 0.1 mV/H |
| Measurement values for human (mmHg) | | |
| 1. Neonatal (3 days old) | 30 | 32 |
| 2. Infant (3 yr. old) | Too active measurement impossible | 35 |
| 3. Adult (healthy) | 41 | 41 |
| 4. Adult (pulmonary edema patient) | 46 | 45 |

A 40 μm thick lens spacer was inserted between the glass electrode and the polymer membrane as a spacer. The sensor was maintained at 43.5°C and the comparative experiment was conducted.

As is clear from Table 2, the sensor according to this embodiment of the invention is far superior to the conventional sensor as regards the noise level, with extremely small drifts, and stable measurement is guaranteed.

As mentioned hereinbefore, according to the transcutaneous $CO_2$ measurement sensor, the following additional advantages are achieved:

a) because the preamplifier is positioned in the glass electrode, the sensor may be reduced to a compact size and may easily be attached to the restricted measurement portion when being used with the premature and neonatal babies;

b) as the preamplifier positioned in the glass electrode is enveloped by the external reference electrode 63, the electrode acts as a shield against the external electric field. The input lead wire for the preamplifier is extremely short, thereby remarkably reducing the static and the electromagnetic induction noise levels;

c) as the preamplifier is positioned in the glass electrode which maintained at a constant temperature, the temperature fluctuation is reduced compared to that of the conventional product. Accordingly, a preamplifier is provided having less gain fluctuation and drift, thereby guaranteeing the stabilized measurement.

## Claims

1. A transcutaneous carbon dioxide measuring assembly which comprises an electrode holder 53 having a glass electrode with an $H^+$ ion sensitive glass membrane 45 at the end of a tubular member 46 made of insulating material, and an external reference electrode 52 located around the circumference of the glass electrode, a membrane holder 55 located around the circumference of the reference electrode having a $CO_2$ permeable polymer membrane 54 disposed at one end thereof, and a membrane fixing means 56 having an aperture through which the gas to be measured is passed to the glass electrode, the membrane fixing means 56 pressing the polymer membrane 54 of the membrane holder 55 onto the glass membrane 45 via a $CO_2$ responsive electrolyte film, the electrode holder, membrane holder and membrane fixing means being three detachable components which are joined to form the said assembly, characterized in that a conductive coating 47 is formed on the inner surface of the glass membrane 45, and a heater member 48 comprising a heater 49 and a heat sensitive element 50 which controls the heater to maintain a predetermined temperature is disposed inside the glass electrode.

2. A transcutaneous carbon dioxide measuring assembly as claimed in claim 1 wherein the membrane holder 55 is supported in a space between the electrode holder and the membrane fixing means 56 and the membrane fixing means 56 is attached to the electrode holder 53 by means of co-operating screw threads formed on the electrode holder and the membrane fixing means.

6

**0 039 243**

3. A transcutaneous carbon dioxide measuring assembly which comprises an electrode holder having a glass electrode with an $H^+$ ion sensitive glass membrane 57 at the end of a tubular member 58 made of an insulating material, and an external reference electrode 63 located around the circumference of the glass electrode, a membrane holder 67 located around the circumference of the reference electrode having a $CO_2$ permeable polymer membrane 68 at one end thereof, and a membrane fixing means 56 having an aperture through which the gas to be measured is passed to the gas electrode, the membrane fixing means 56 pressing the polymer membrane 68 of the membrane holder 67 onto the glass membrane 57 via a $CO_2$ responsive electrolyte film 27, the electrode holder, membrane holder and membrane fixing means being three detachable components which are joined to form the said assembly, characterized in that a conductive coating 59 is formed on the inner surface of the glass membrane 57 and a preamplifier 60 is housed inside the glass electrode, the preamplifier 60 being connected to the external reference electrode 63 and to the main measuring device.

4. A transcutaneous carbon dioxide measuring assembly as claimed in claim 3 wherein the preamplifier is connected to the conductive coating 59.

**Revendications**

1. Dispositif de mesure transcutanée de dioxyde de carbone, qui comprend un porte-électrode 53 comportant une électrode de verre pourvue d'un diaphragme en verre 45 sensible aux ions $H^+$, à l'extrémité d'un élément tubulaire 46 réalisé en une matière isolante, ainsi qu'une électrode de référence extérieure 52 placée autour de la circonférence de l'électrode de verre, un porte-diaphragme 55 placé autour de la circonférence de l'électrode de référence et portant un diaphgrame en polymère 54 perméable au $CO_2$ et disposé à l'une des extrémités de ce porte-diaphragme, et un organe de fixation de diaphragme 56 présentant une ouverture à travers laquelle le gaz à mesurer circule en direction de l'électrode de verre, l'organe de fixation de diaphragme 56 pressant le diaphragme en polymère 54 du porte-diaphragme 55 contre le diaphragme en verre 45, avec interposition d'un film d'électrolyte sensible au $CO_2$, le porte-électrode, le porte-diaphragme et l'organe de fixation de diaphragme étant trois composants démontables qui sont assemblés pour former ledit dispositif, caractérisé en ce qu'un revêtement conducteur 47 est formé sur la surface intérieure du diaphragme en verre 45 et un organe de chauffage 48, comprenant un élément chauffant proprement dit 49 et un élément thermosensible 50 qui assure une régulation de l'élément chauffant pour maintenir une température prédéterminée, est disposé à l'intérieur de l'électrode de verre.

2. Dispositif de mesure transcutanée de dioxyde de carbone tel que défini dans la revendication 1, dans lequel le porte-diaphragme 55 est supporté dans un espace situé entre le porte-électrode 53 et l'organe de fixation de diaphragme 56, et l'organe de fixation de diaphragme 56 est solidairisé au porte-électrode 53 au moyen de filetages complémentaires formés sur le porte-électrode et sur l'organe de fixation de diaphragme.

3. Dispositif de mesure transcutanée de dioxyde de carbone, qui comprend un porte-électrode comportant une électrode de verre pourvue d'un diaphgrame en verre 57 sensible aux ions $H^+$, à l'extrémité d'un élément tubulaire 58 réalisé en une matière isolante, ainsi qu'une électrode de référence extérieure 63 placée autour de la circonférence de l'électrode de verre, un porte-diaphragme 67 placé autour de la circonférence de l'électrode de référence et portant un diaphgragme en polymère 68 perméable au $CO_2$ à l'une de ses extrémités, et un organe de fixation de diaphragme 56 présentant une ouverture à travers laquelle le gaz à mesurer circule en direction de l'électrode de verre, l'organe de fixation de diaphragme 56 pressant le diaphragme en polymère 68 du porte-diaphragme 67 contre le diaphragme en verre 57, avec interposition d'un film d'électrolyte 27 sensible au $CO_2$, le porte-électrode, le porte-diaphragme et l'organe de fixation de diaphragme étant trois composants démontables qui sont assemblés pour former ledit dispositif, caraactérisé en ce qu'un revêtement conducteur 59 est formé sur la surface intérieure du diagrame en verre 57 et un pré-amplificateur 60 est logé à l'intérieur de l'électrode de verre, le pré-amplificateur 60 étant connecté à l'électrode de référence extérieure 63 et à l'appareil de mesure principal.

4. Dispositif de mesure transcutanée de dioxyde de carbone tel que défini dans la revendication 3, dans lequel le pré-amplificateur est connecté au revêtement conducteur 59.

**Patentansprüche**

1. Anordnung zum Messen transkutan austretenden Kohlendioxids, umfassend einen Elektrodenhalter (53) mit einer Glaselektrode, die eine $H^+$-ionenempfindliche Glasmembran (45) am Ende eines Rohrelements (46) aus Isoliermaterial aufweist, und einer um den Umfang der Glaselektrode herum angeordneten externen Bezugselektrode (52), einen um den Umfang der Bezugselektrode herum angeordneten Membranhalter (55) mit einer an seinem einen Ende vorgesehenen, $CO_2$-durchlässigen polymeren Membran (54) sowie eine Membranfixiereinrichtung (56) mit einer Bohrung, durch welche das zu messende Gas zur Glaselektrode hindurchtritt, wobei die Membranfixiereinrichtung (56) die polymere Membran (54) des Membranhalters (55) über einen auf $CO_2$ ansprechenden Elektrolytfilm gegen die Glasmembran (45) andrückt, (und) der Elektrodenhalter, der Membranhalter und die Membranfixier-

7

einrichtung drei trennbare Bauteile darstellen, die zur Bildung der Anordnung miteinander verbunden oder vereinigt sind, dadurch gekennzeichnet, daß an der Innenfläche der Glasmembran (45) ein leitfähiger Überzug (47) ausgebildet ist und innerhalb der Glaselektrode eine Heizeinheit (48) aus einem Heizelement (49) und einem wärmeempfindlichen Element (50), welches das Heizelement zur Aufrechterhaltung einer vorbestimmten Temperatur ansteuert, angeordnet ist.

2. Anordnung zum Messen transkutan austretenden Kohlendioxids nach Anspruch 1, dadurch gekennzeichnet, daß der Membranhalter (55) in einem Raum zwischen dem Elektrodenhalter (53) und der Membranfixiereinrichtung (56) gehaltert ist und die Membranfixiereinrichtung (56) am Elektrodenhalter (53) mittels zusammenwirkender, am Elektrodenhalter und an der Membranfixiereinrichtung ausgebildeter Gewindegänge angebracht ist.

3. Anordnung zum Messen transkutan austretenden Kohlendioxids, umfassend einen Elektrodenhalter mit einer Glaselektrode, die eine $H^+$-ionenempfindliche Glasmembran (57) am Ende eines Rohrelements (58) aus einem Isoliermaterial aufweist, und einer um den Umfang der Glaselektrode herum angeordneten externen Bezugselektrode (63), einen um den Umfang der Bezugselektrode herum angeordneten Membranhalter (67) mit einer an seinem einen Ende vorgesehenen, $CO_2$-durchlässigen polymeren Membran (68) sowie eine Membranfixiereinrichtung (56) mit einer Bohrung, durch welche das zu messende Gas zur Gas-bzw. Glaselektrode hindurchtritt, wobei die Membranfixiereinrichtung (56) die polymere Membran (68) des Membranhalters (67) über einen auf $CO_2$ ansprechenden Elektrolytfilm (27) gegen die Glasmembran (57) andrückt, (und) der Elektrodenhalter, der Membranhalter und die Membranfixiereinrichtung drei trennbare Bauteile darstellen, die zur Bildung der Anordnung miteinander verbunden oder vereinigt sind, dadurch gekennzeichnet, daß an der Innenfläche der Glasmembran (57) ein leitfähiger Überzug (59) ausgebildet ist und im Inneren der Glaselektrode ein Vorverstärker (60) untergebracht ist, der mit der externen Bezugselektrode (63) und der Hauptmeßvorrichtung verbunden ist.

4. Anordnung zum Messen transkutan austretenden Kohlendioxids nach Anspruch 3, dadurch gekennzeichnet, daß der Vorverstärker an den leitfähigen Überzug (59) angeschlossen ist.

0 039 243

FIG. 1.

FIG. 2.

FIG. 3.

## FIG. 4.